# EUROPEAN PATENT APPLICATION

(11) **EP 4 012 044 A1**
(43) Date of publication of application: **15.06.2022**
(21) Application number: 20212878.1
(22) Date of filing: 09.12.2020
(51) Int. Cl.: C12Q 1/37, C12Q 1/70, G01N 33/569, G01N 33/573

(54) **METHOD FOR DETECTING A VIRUS**

(71) Applicant: Qualizyme Diagnostics GmbH & Co KG, 8010 Graz (AT)
(72) Inventor: SIGL, Eva, 8010 Graz (AT); HEINZLE, Andrea, 8010 Graz (AT); LUSCHNIG, Daniel, 8010 Graz (AT); NOVAK, Sandra, 8010 Graz (AT); WINKLER, Sonja, 8010 Graz (AT); GAMERITH, Clemens, 8010 Graz (AT)
(74) Representative: Schwarz & Partner Patentanwälte GmbH

(57) **Abstract**

The present invention relates to a method for detecting a receptor binding virus in a sample, wherein said receptor exhibits enzymatic properties, com-prising the steps of:
a) incubating said sample with said receptor to form a sample-receptor mixture;
b) contacting the mixture of step a) with a substrate cat-alyzable by said receptor;
c) determining if said substrate is converted by said receptor; and
d) detecting a receptor binding virus in said sample if said substrate is converted by said receptor to a lower extent compared to a reference sample.

## Description

### TECHNICAL FIELD

The present invention relates to a rapid diagnostic test for detecting a virus.

### BACKGROUND ART

Rapid diagnosis of viral infections is crucial, especially in times of a pandemic caused, for instance, by the coronavirus SARS-CoV-2.

Several testing strategies for viruses like SARS-CoV-2 are currently available on the market, including polymerase chain reaction (PCR) and antibody tests. PCR tests are based on the replication of genetic material and are very reliable. However, these tests require special laboratory equipment and additionally suffer from long analysis times and high costs. Antibody tests detect antibodies produced in response to a viral infection and may therefore not be suitable to detect early stages of the infection. In addition, both PCR and antibody tests require medical personnel to obtain a sample from the patient and a skilled technician to analyse the samples.

Thus, there is still a need for a rapid and cheap test providing information about an infection with a virus like SARS-CoV-2 within minutes, which test is ideally performed without the aid of specially trained personnel.

The objective of the present invention is to provide a test which fulfils these criteria and additionally has high diagnostic sensitivity and specificity.

### SUMMARY OF THE INVENTION

The present invention relates to a method for detecting a receptor binding virus in a sample, wherein said receptor exhibits enzymatic properties, comprising the steps of:
a) incubating said sample with said receptor to form a sample-receptor mixture;
b) contacting the mixture of step a) with a substrate catalyzable by said receptor;
c) determining if said substrate is converted by said receptor; and
d) detecting a receptor binding virus in said sample if said substrate is converted by said receptor to a lower extent compared to a reference sample.

The method according to the present invention is suitable to detect any virus, which uses an enzyme as a receptor molecule.

Examples for such viruses comprise coronaviruses, herpesviruses, such as human gammaherpesvirus 4 and human alphaherpesvirus 3, and avian hepatitis B virus. Among coronaviruses, the severe acute respiratory syndrome-related coronaviruses SARS-CoV-1 and SARS-CoV-2, the Middle East respiratory syndrome-related coronavirus (MERS-CoV), human coronavirus 229 E, transmissible gastroenteritis coronavirus and porcine epidemic diarrhea virus use an enzyme as a receptor molecule.

Preferably, the virus to be detected according to the present invention belongs to the coronavirus family, in particular SARS-CoV-2, which use angiotensin-converting enzyme 2 (ACE2) as a receptor.

Surprisingly, it turned out that the inventive method enables rapid and accurate detection of said virus.

Detection of said virus may be performed colorimetrically. Within a short time span, a clear result based on colour development or colour erasure provides information on whether the tested sample comprises said virus (positive test) or not (negative test).

The present invention further relates to a kit detecting a receptor binding virus in a sample comprising:
a) a receptor binding said virus, wherein said receptor exhibits enzymatic properties;
b) a substrate catalyzable by said receptor;
c) optionally a second enzyme converting the product of the conversion of said substrate by said receptor;
d) at least one compound undergoing a colour change if said substrate is converted by said receptor.

The present invention also relates to a test strip for detecting a receptor binding virus, comprising a solid support comprising said receptor, a substrate catalyzable by said receptor, a compound undergoing a colour change if said substrate is converted by said receptor, and optionally a second enzyme converting the product of the conversion of said substrate by said receptor.

The present invention also relates to a test strip for detecting a receptor binding virus, comprising a solid support comprising said receptor, a substrate catalyzable by said receptor, a compound undergoing a colour change if said substrate is converted by said receptor, and optionally a second enzyme converting the product of the conversion of said substrate by said receptor.

### SHORT DESCRIPTION OF THE FIGURES

Fig. 1 shows an analysis scheme for detecting SARS-CoV-2 via the colour change of a pH indicator.
Fig. 2 shows an analysis scheme for detecting SARS-CoV-2 via the colour change of a redox agent (FeCl₃).
Fig. 3a shows a scheme of a lateral flow test strip based on a pH value shift.
Fig. 3b shows a plot of the in-vitro diagnostic (IVD) rapid test device for SARS-CoV-2 of Fig. 3a.
Fig. 4a shows a scheme of a lateral flow test strip based on the colour change of a redox agent.
Fig. 4b shows a plot of the IVD rapid test device for SARS-CoV-2 of Fig. 4a.
Fig. 5a shows a scheme of a combined liquid assay and a solid phase assay based on the colour change of a redox agent (FeCl₃) for a positive test.
Fig. 5b shows a scheme of a combined liquid assay and a solid phase assay based on the colour change of a redox agent (FeCl₃) for a negative test.
Fig. 6a shows a scheme of a liquid assay based on the colour change of a redox agent (FeCl₃) for a positive test.
Fig. 6b shows a liquid assay based on the colour change of a redox agent (FeCl₃) for a negative test.

### DESCRIPTION OF EMBODIMENTS

According to the present invention, a virus binding to a receptor ("receptor binding virus") is detected via its receptor protein, which receptor protein has enzymatic properties. The conversion of a substrate by said receptor is reduced or prevented by the binding of said virus to the receptor.

In a first step of the inventive method, the sample potentially comprising the virus is incubated with the receptor. Then, a substrate is added, which substrate is catalyzable by the receptor. In case the sample contains said virus, the receptor will be blocked and the substrate will not be converted or will be converted to a lower extent by the receptor because the binding of the virus to the receptor interferes with its enzymatic activity. In case the sample does not contain any virus, the receptor functions as an enzyme and the substrate will be converted. Thus, it is determined if said substrate is converted or not in a third step of the inventive method. In a fourth step, the conversion of said substrate in the presence of the sample potentially comprising the virus is compared to the conversion of the substrate in the presence of a reference sample.

The conversion of a substrate by said receptor may be determined colorimetrically, chromatographically or fluorometrically. Preferably, the conversion of said substrate is coupled to a chromogenic reaction indicating if said substrate is converted or not. Thus, if the substrate is converted, a colour change is observed, corresponding to a sample substantially free of said virus (negative sample). If the substrate is not converted (no colour change) or converted to a lower extent, the sample comprises the virus (positive sample).

The colorimetric detection has the advantage of providing a quick and simple test result (positive or negative) .

Preferably, the reference sample is substantially free of said virus. The term "substantially free" as used herein means that the reference sample contains no or only traces of viral material, whereby it is particularly preferred using a reference sample comprising no detectable viruses.

In case of a positive test result, a further reference sample may be measured to ensure that the positive result is indeed caused by the virus to be detected, such as SARS-CoV-2. Thus, as a further reference sample, the sample which has caused a positive test result may be used, additionally comprising an antibody specific for the virus to be detected. If the virus is indeed present in the sample, it will bind to the antibody. In particular, an antibody blocking the binding site of the virus to the receptor is chosen. Binding of said virus to the antibody will thus inhibit binding of the virus to the receptor enzyme and therefore, the substrate added to the receptor will be converted in the same way as in a sample substantially free of said virus. Instead of the antibody, an antibody derivative comprising the viral binding region of said antibody may be used. The use of an antibody specific for the virus to be detected ensures that a positive test result is indeed caused by the virus to be detected. This strategy guarantees that there will be no virus-receptor interaction resulting in a false positive result.

According to a preferred embodiment of the present invention, said receptor is selected from the group consisting of angiotensin-converting enzyme 2 (ACE2), dipeptidyl peptidase-4 (DPP4), aminopeptidase N (AP-N), complement receptor type 1 (CR1), carboxypeptidase and insulin-degrading enzyme (IDE) .

Viruses binding to receptors having enzymatic activities are well-known in the art. However, it is particularly preferred that said receptor binding virus is selected from the group consisting of a severe acute respiratory syndrome (SARS)-related coronavirus, Middle East respiratory syndrome (MERS)-related coronavirus, human coronavirus 229 E, transmissible gastroenteritis coronavirus, porcine epidemic diarrhea virus, human gammaherpesvirus 4, avian hepatitis B virus and human alphaherpesvirus 3 (HHV-3).

If said virus is a SARS-related coronavirus selected from the group consisting of SARS-CoV-1 and SARS-CoV-2, said receptor is ACE2. If said virus is MERS-CoV, said receptor is DPP4. If said virus is human coronavirus 229 E, transmissible gastroenteritis coronavirus or porcine epidemic diarrhea virus, said receptor is AP-N. If said virus is human gammaherpesvirus 4, which is also referred to as "Epstein-Barr virus (EBV)", said receptor is CR1. If said virus is avian hepatitis B virus, said receptor is carboxypeptidase D. If said virus is human alphaherpesvirus 3 (HHV-3), which is also referred to as "varicella-zoster virus (VZV)", said receptor is IDE.

In a particularly preferred embodiment of the present invention, said receptor is ACE2 and said virus is SARS-CoV-2.

In an embodiment of the present invention, the substrate is converted by the receptor to at least one first product, wherein said at least one first product is detected by converting said at least one first product to at least one second product by a second enzyme, wherein said at least one second product is detected colorimetrically, chromatographically or fluorometrically. Thus, said at least one first product may function as a substrate in a second conversion reaction catalysed by a second enzyme From said second conversion reaction one or more products may be formed, herein referred to as the "at least one second product". The at least one second product may be detected colorimetrically, chromatographically or fluorometrically. Preferably, detection is performed colorimetrically.

Colorimetric detection of a second product may involve the colour change of a redox agent or the colour change of a pH indicator. Thus, if the second product is formed (negative sample), a redox agent may undergo a colour change, for example by the formation of a coloured complex between the redox agent and the second product. A pH indicator may undergo a colour change due to a change in the pH value upon formation of a second product. If the second product is not formed (positive sample), preferably no colour change occurs.

Said substrate which is converted to the first product must be a substrate convertible by the receptor according to the present invention. If said virus is SARS-CoV-2 and said receptor is ACE2, said substrate is a substrate of ACE2. Said substrate may for example be a physiological or a non-physiological substrate of ACE2.

The SARS-CoV-2 virus gains access to its host cell through its S protein ("spike" protein), which is a glycosylated protein of the virus envelope. The S-protein consists of 2 subunits, which are designated S1 and S2. The S1 subunit is the receptor binding domain (RBD) that binds to the cellular receptor ACE2. ACE2 is a metalloprotease, which plays an important role in the RAAS (renin-angiotensin-aldosterone system). In the RAA-System, the decapeptide angiotensin 1-10 ("Ang I" or "Ang 1-10") is cleaved from the precursor molecule angiotensinogen (452 AA), by renin. The inactive AngI is converted to the vasoconstrictive octapeptide angiotensin 1-8 ("Ang II") by the dipeptidyl carboxypeptidase ACE (angiotensin converting enzyme). Ang II fulfils its physiological function by binding to the AT1 and AT2 receptors. The metallocarboxypeptide ACE2, a transmembrane protein, cleaves the C-terminal amino acid (aa) leucine (Leu) from Ang I and generates the nonapeptide Ang 1-9, which physiological function is unclear. In addition, ACE2 cleaves the C-terminal phenylalanine (Phe) from the octapeptide Ang II, leading to the vasodilatory heptapeptide Ang 1-7. ACE2 thus limits the vasoconstrictive octapeptide Ang II by degradation to the vasodilatory heptapeptide Ang 1-7.

Thus, Ang II (amino acid sequence: DRVYIHPF, SEQ ID No. 2) or a derivative thereof may be used as a substrate convertible by ACE2 according to the present invention. The derivative of ACE2 may comprise a tag at the N-terminal amino acid, or any further amino acid.

In a preferred embodiment of the present invention, said substrate is a peptide comprising or consisting of the amino acid sequence (X₁)ₘDRVYIHP(X₂)ₙ (SEQ ID No. 1), wherein X₁ is an N-terminal tag, preferably selected from the group consisting of a cholesteryl compound, an aromatic compound, a heterocyclic compound and a heteroaromatic compound, and wherein X₂ is selected from the group consisting of phenylalanine (F), a fluorescent label and a chromophoric label, and wherein m is 0 or 1 and n is 0 or 1. According to a particularly preferred embodiment at least one of m or n is 1.

The term "fluorescent label" herein refers to an organic molecule having a fluorescent moiety, which organic molecule is covalently linked, optionally via a linker moiety, to the amino acid P in SEQ ID No. 1. Preferably, the fluorescent label is 7-amino-4-methylcoumarin (AMC) or a derivative thereof.

The term "chromophoric label" herein refers to an organic molecule absorbing visible light, which organic molecule is covalently linked, optionally via a linker moiety, to the amino acid P in SEQ ID No. 1. Preferably, the chromophoric label used in the present invention is para-nitroaniline.

If said substrate is a peptide comprising SEQ ID No. 1, said peptide is a sequence preferably consisting of 9 to 15 amino acids.

Preferably, the substrate is a peptide consisting of the amino acid sequence DRVYIHPF (SEQ ID No. 2), or said substrate is a peptide consisting of SEQ ID No. 1, wherein X₂ is F. Preferably, phenylalanine (F) is produced if the substrate is converted by ACE2.

Thus, in a preferred embodiment, the first product comprises phenylalanine (F).

Said first product may be converted to at least one second product by a second enzyme.

Specifically, said second enzyme may be a phenylalanine processing enzyme (PPE), preferably selected from the group consisting of L-amino acid deaminase (LAAD), L-amino acid oxidase (LAAO), phenylalanine ammonia lyase (PAL) and L-amino acid dehydrogenase.

If the first product comprises phenylalanine, and the second enzyme is LAAD, the second product comprises ammonia and phenylpyruvate. If the first product comprises phenylalanine, and the second enzyme is LAAO, the second product comprises ammonia, phenyl pyruvate and hydrogen peroxide. If the first product comprises phenylalanine, and the second enzyme is L-amino acid dehydrogenase, the second product comprises ammonium ions, phenyl pyruvate and NADH. If the first product comprises phenylalanine, and the second enzyme is PAL, the second product comprises ammonia and trans-cinnamic acid.

Preferably, said second product comprises ammonia (NH₃) and/or phenyl pyruvate and/or hydrogen peroxide (H₂O₂).

According to one embodiment of the present invention, said ammonia is detected by the colour change of a pH indicator. Said pH indicator is preferably a triarylmethane dye, preferably selected from the group consisting of bromothymol blue and bromocresol purple.

According to a preferred embodiment of the present invention, the second product comprises phenylpyruvate, which is detected by the colour change of a redox agent. Said redox agent may be any inorganic or organic chemical compound which develops a colour when undergoing a redox reaction or a complexation reaction. The skilled person is well aware of suitable redox agents. According to a preferred embodiment of the present invention, said redox agent is FeCl₃. Reaction of FeCl₃ with phenyl pyruvate results in the formation of a green complex.

According to another preferred embodiment, the second product comprises hydrogen peroxide. Said hydrogen peroxide may be detected by adding a peroxidase and a compound, which is a substrate of peroxidase and undergoes a colour change if oxidized by hydrogen peroxide in a reaction catalysed by peroxidase. Said compound may be a phenolic compound like Guaiacol (1-hydroxy-2-methoxybenzene), 3,4-diaminobenzoic acid and derivatives thereof. According to a preferred embodiment, said compound is 3,3',5,5'-tetramethylbenzidine (3,3',5,5'-tetramethyl[1,1'-biphenyl]-4,4'-diamine, TMB) or a derivative thereof. The advantage of detecting hydrogen peroxide with peroxidase and TMB is that a solid phase comprising peroxidase and TMB can be provided.

The method according to the present invention may be performed in liquid phase and/or on a solid phase.

Preferably, the conversion of the substrate by the receptor to the first product and the conversion of said first product to the second product is performed in liquid phase. Preferably, the second product is detected using a solid support comprising a redox agent and optionally a trap agent. The solid support may alternatively comprise a pH indicator. The solid support may be made of cellulose. Said solid support may be in the form of a filter strip.

Preferably, the redox agent is applied at a zone within the upper part of said filter strip. Optionally, a trap agent is applied above said redox agent. In a preferred embodiment, the second product comprises phenyl pyruvate and the redox agent is FeCl₃. If a trap agent is used, the trap agent is preferably a quaternary ammonium compound, such as a tetraalkyl ammonium compound of general formula R₄N⁺X⁻, wherein R is an alkyl group and X is any suitable anion, preferably a halide ion, even more preferably chloride or bromide. The quaternary ammonium compound is able to bind a negatively charged compound.

The quaternary ammonium compound is preferably selected from the group consisting of tetraoctylammonium chloride, tetraoctylammonium bromide, methyltrioctylammonium chloride, methyl trioctylammonium bromide, tetradodecylammonium chloride, tetradodecylammonium bromide, tetrapentylammonium chloride, tetrapentylammonium bromide, tetrahexylammonium chloride, tetrahexylammonium bromide, tetrapropylammonium chloride, tetrapropylammonium bromide, tetrabutylammonium chloride and tetrabutylammonium bromide. Even more preferably, the quaternary ammonium compound is tetra-n-octylammonium bromide.

The filter strip may be put into the liquid sample and the sample is pulled up until it reaches the zone of the redox agent. In case the second product is phenyl pyruvate and the redox agent is FeCl₃, a green complex is formed which may be trapped by a quaternary ammonium compound, such as tetra-n-octylammonium bromide, resulting in a brown band on the filter strip. The formation of a brown band indicates that phenylpyruvate has been formed and thus no virus is present in the sample (negative test). If no band is formed, the test is positive (sample comprising virus).

The present invention further relates to a kit for detecting a receptor binding virus in a sample. Said kit comprises a receptor, a substrate of said receptor, and a compound undergoing a colour change. Preferably, said compound is a redox agent such as FeCl₃. Even more preferably, the redox agent is positioned on a solid support, preferably a filter strip, as described herein.

If the first product is not detected directly, the inventive kit comprises a second enzyme to produce the second product.

In another embodiment, the inventive method may be entirely performed on solid phase. Thus, a test strip for detecting a receptor binding virus is disclosed herein, on which test strip said receptor is positioned in a first zone, and a substrate catalyzable by said receptor is positioned in a second zone above said first zone. Optionally, the second enzyme is positioned above the second zone. In a final zone, the compound undergoing a colour change is positioned.

Preferably, said test strip is a lateral flow test strip.

### DETAILED DESCRIPTION OF THE FIGURES

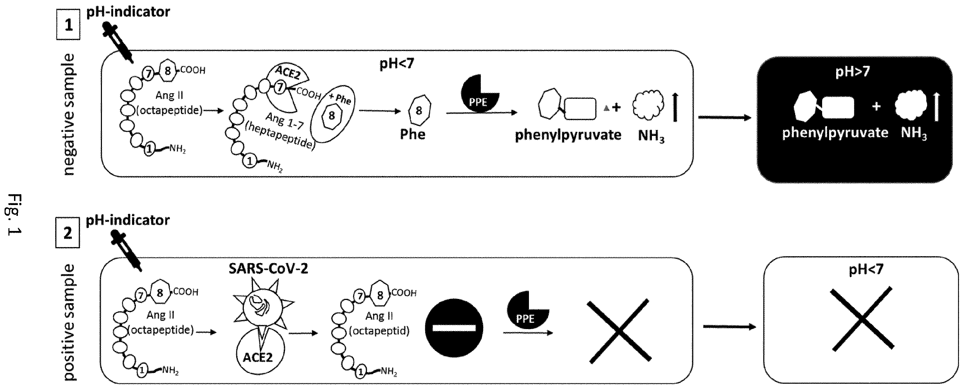

**Fig. 1****:** Detection of SARS-CoV-2 via the colour change of a pH indicator. (1): Reaction sequence for a negative sample. Both enzyme reactions can take place. The 2^{nd} reaction produces NH₃, which causes the colour change of the solution through the addition of a proper pH-indicator. (2): Reaction sequence for a positive sample. The 1^{st} reaction (ACE2 - the physiological) does not take place since the virus has bound to ACE2 and blocks or decelerates the physiological function of ACE2. Thus, no, or less substrate (Phe) is formed for the 2^{nd} reaction. The 2^{nd} reaction (PPE) cannot take place - no NH₃ is produced - no pH shift occurs (no colour change in the solution).

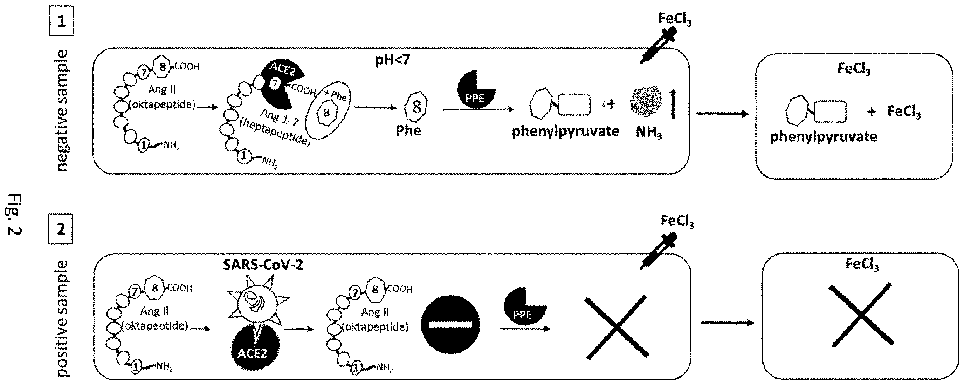

**Fig. 2****:** Detection of SARS-CoV-2 via the colour change of FeCl₃. (1): Reaction sequence for a negative sample. Both enzyme reactions can take place. In the second reaction, phenylpyruvate is formed in addition to the by-products (e.g.NH₃ and or H₂O₂), which reacts to a green-coloured complex when FeCl₃ is added. (2) Reaction sequence for a positive sample. The first reaction (physiological) does not take place, because the virus has docked on ACE2 and blocks, or drastically decreases, the physiological function of ACE2. Thus, no, or less substrate is formed for the 2^{nd} enzyme reaction. The second reaction cannot take place - no phenylpyruvate is formed - no green complex can be formed when FeCl₃ is added (no colour change in the solution).

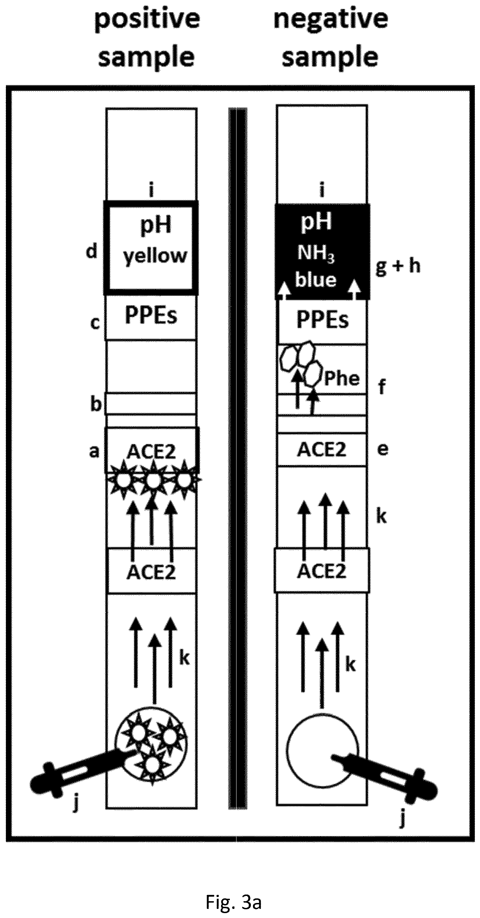

**Fig. 3a****:** Strategy 1 - "lateral flow test strip" based on a pH value shift. Reaction scheme for a positive sample (left) and a negative sample (right). Positive sample (left): The 1^{st} enzyme reaction (physiological) does not take place since the virus has docked to ACE2 and thus, the physiological function of ACE2 (protease) is blocked (a). The ACE2-SARS-CoV-2-complex migrates to the substrate (octapeptide, Ang II) . No substrate (octapeptide) is converted to the heptapeptide (Ang 1-7) + phenylalanine (b). The 2^{nd} enzyme reaction (PPE) cannot take place - no NH₃ is formed (c) - there is no pH shift - the colored pH indicator area remains yellow (d). **Negative sample (right):** Both enzyme reactions can take place. The free ACE2 (e) migrates to the substrate (octapeptide, Ang II) and converts the octapeptide to the heptapeptide + Phe (f). In the second enzyme reaction, Phe is converted to phenylpyruvate and NH₃ by the PPE (g). The released NH₃ induces the color change of the pH indicator area - the colored pH indicator area turns blue (h). ACE2 (angiotensin converting enzyme 2), Ang II (angiotensin II -> octapeptide), Ang 1-7 (angiotensin 1-7 -> heptapeptide), Phe (phenylalanine), PPE (Phenylalanine Processing Enzyme); (i) detection window; (j) sample application area - left: negative sample (-CoV), right: positive sample +CoV; (k) flow/direction of sample movement

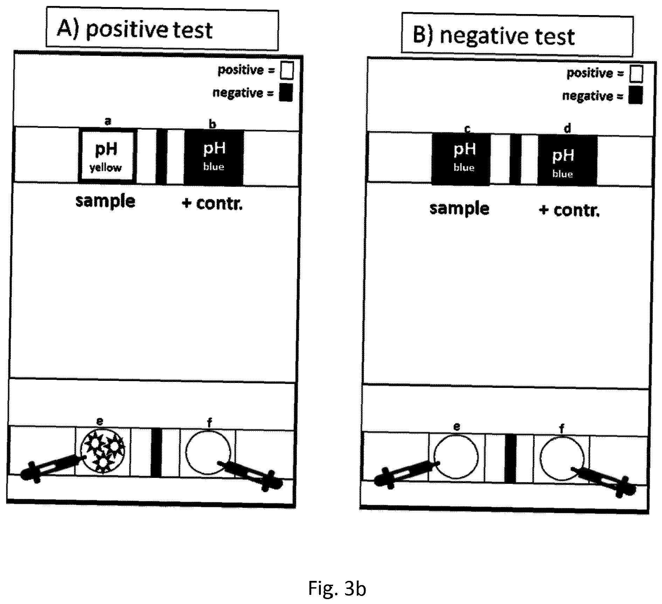

**Fig. 3b****:** Plot of the IVD rapid test device for SARS-CoV-2. A): Positive test result: The left detection window (a) shows a different colour than the right detection window (b). B): Negative test result: The left detection window (c) shows the same colour as the right detection window (d). Detection windows are denoted with a to d. Sample application areas are denoted with "e", control application areas (buffer or 0.9% NaCl) are denoted with "f".

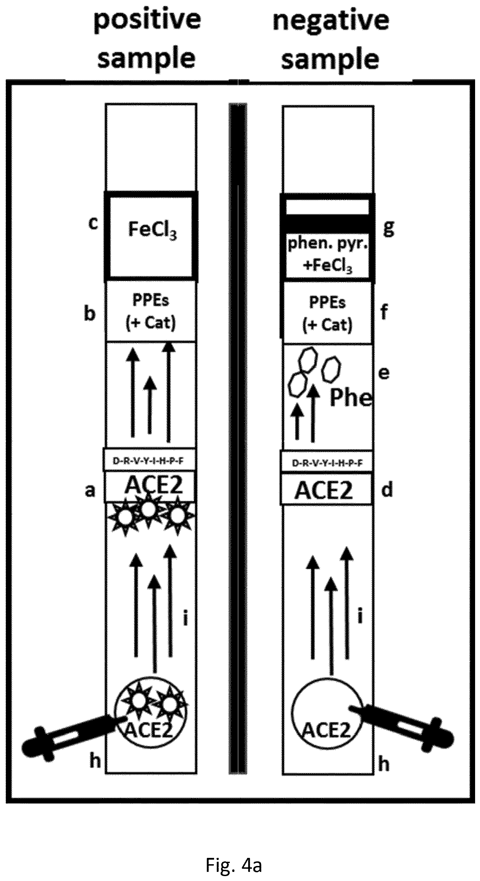

**Fig. 4a****:** Strategy 1 - "lateral flow test strip" based on FeCl₃ detection. Reaction scheme for a positive sample (left) and a negative sample (right). Positive sample (left): The 1^{st} enzyme reaction (physiological) does not take place since the virus has docked to ACE2 and thus, the physiological function of ACE2 (protease) is blocked. The ACE2-SARS-CoV-2-complex migrates to the substrate (a), the octapeptide, Ang II. No substrate (octapeptide, DRVYIHPF) is converted to the heptapeptide (Ang 1-7) + phenylalanine. The 2^{nd} enzyme reaction (PPE) cannot take place (b) since no phenylpyruvate (and by-products NH_{3/}H₂O₂) were formed - there is no color-development (green/brown band). The indicator area of the detection window remains empty, meaning a white area containing FeCl₃ (c). Negative sample (right): Both enzyme reactions can take place. The free ACE2 migrates to the substrate, the octapeptide, DRVYIHPF (d) and converts the octapeptide to the heptapeptide Ang 1-7 and Phe (e). During the second enzyme reaction (f), Phe is deaminated to phenylpyruvate (and by-products NH₃/H₂O₂). The released phenylpyruvate induces the color development with FeCl₃ within the indicator area of the detection window (g), containing the trap (tetra-n-octylammonium bromide) . Since the protease function of ACE2 was not blocked by binding of the Corona virus, the enzyme cascade proceeded, finally resulting in the formation of a brown/green band. ACE2 (angiotensin converting enzyme 2), Ang II (angiotensin II -> octapeptide), Ang 1-7 (angiotensin 1-7 -> heptapeptide), Phe (phenylalanine), PPE (Phenylalanine Processing Enzyme). (h) displays the application area of the sample (left) and the positive control (right). (i) designates the lateral flow direction of the applied liquid.

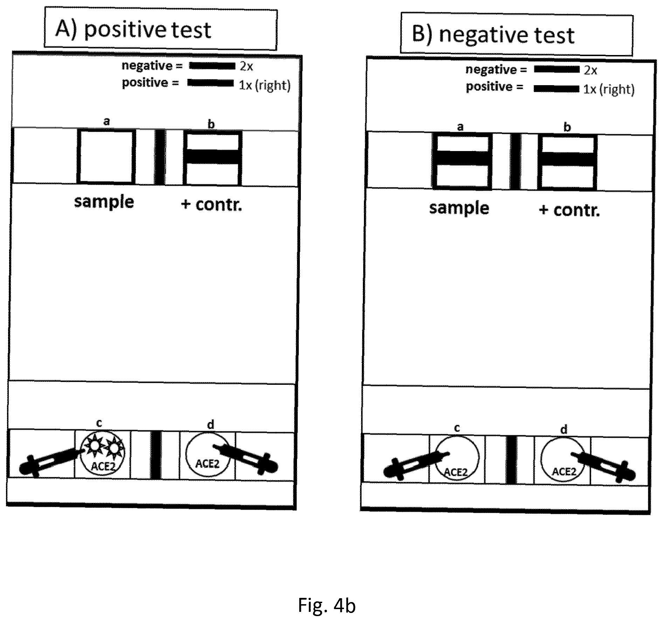

**Fig. 4b****:** Plot of the IVD rapid test device for SARS-CoV-2. A: Positive test result: The left detection window (a) is empty and white - no band is shown, the right detection window (b) displays a brown/green band. B: Negative test result: Both detection windows left and right (a and b) show a brown/green band. The right detection window (b) must display the brown/green band (positive control), otherwise the test is invalid. (c and d) display the application area of the sample (left, c) and the positive control (right, d).

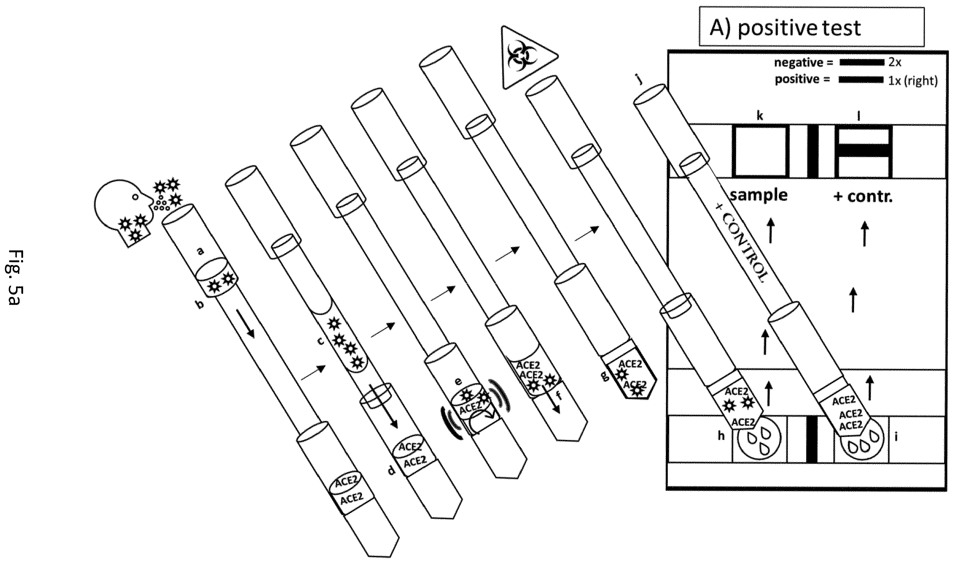

**Fig. 5a****:** Combination of a liquid assay and a solid phase detection mode (lateral flow) using the phenylpyruvate - FeCl₃ strategy. A SARS-CoV-2 positive test result is shown. (a) An infected sample is applied into the application chamber. (b) The application chamber is locked until the sample is applied. (c) The application chamber is unlocked to enable the mixture of sample and reaction-mix. (d) The reaction chamber, containing initially only the pure reaction-mix, is also locked to prevent leakage. (e) Shaking of the device enables the mixture of sample and reaction-mix. (f) The mixture is incubated for 5-10 minutes to enable the docking process of the viral S1 (spike) protein to its receptor hACE2. After incubation, the reaction chamber is unlocked to apply the test-liquid onto the solid phase (lateral flow device). (h) Application of the test-liquid onto the sample lane. (i) Application of the positive control (pure reaction-mix containing no SARS-CoV-2) onto the + control line. (k) Detection window of the sample line - no band denotes a SARS-CoV-2 positive sample. (i) Detection window of the + control line - a brown/green band denotes a valid test result.

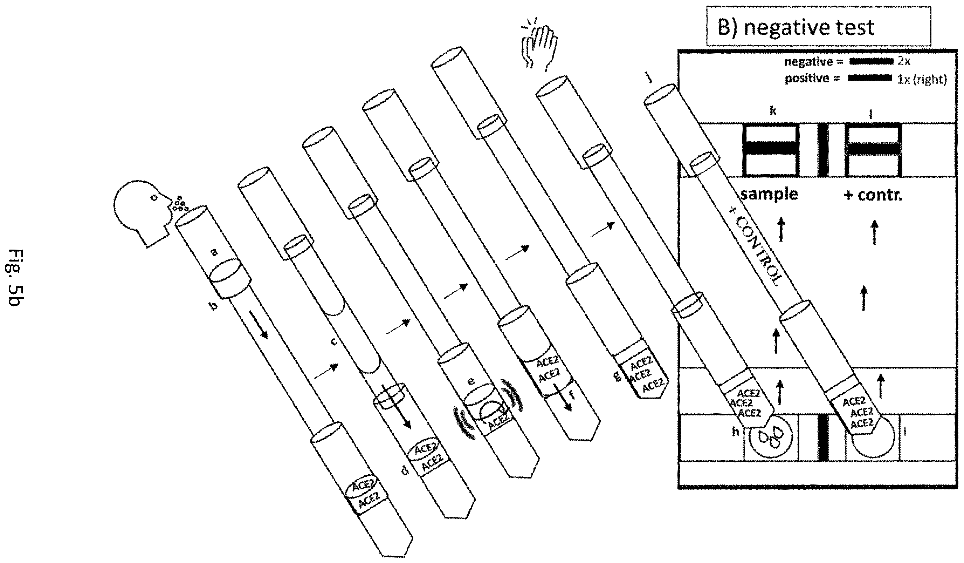

**Fig. 5b****:** Combination of a liquid assay and a solid phase detection mode (lateral flow) using the phenylpyruvate - FeCl₃ strategy. A SARS-CoV-2 negative test result is shown. (a) An non-infected sample is applied into the application chamber. (b) The application chamber is locked until the sample is applied. (c) The application chamber is unlocked to enable the mixture of sample and reaction-mix. (d) The reaction chamber, containing initially only the pure reaction-mix, is also locked to prevent leakage. (e) Shaking of the device enables the mixture of sample and reaction-mix. (f) The mixture is incubated for 5-10 minutes to enable the docking process of the viral S1 (spike) protein to its receptor hACE2, if available. After incubation, the reaction chamber is unlocked to apply the test-liquid onto the solid phase (lateral flow device). (h) Application of the test-liquid onto the sample lane. (i) Application of the positive control (pure reaction-mix containing no SARS-CoV-2) onto the + control line. (k) Detection window of the sample line - a brown/green band denotes a SARS-CoV-2 negative sample. (i) Detection window of the + control line - a brown/green band denotes a valid test result.

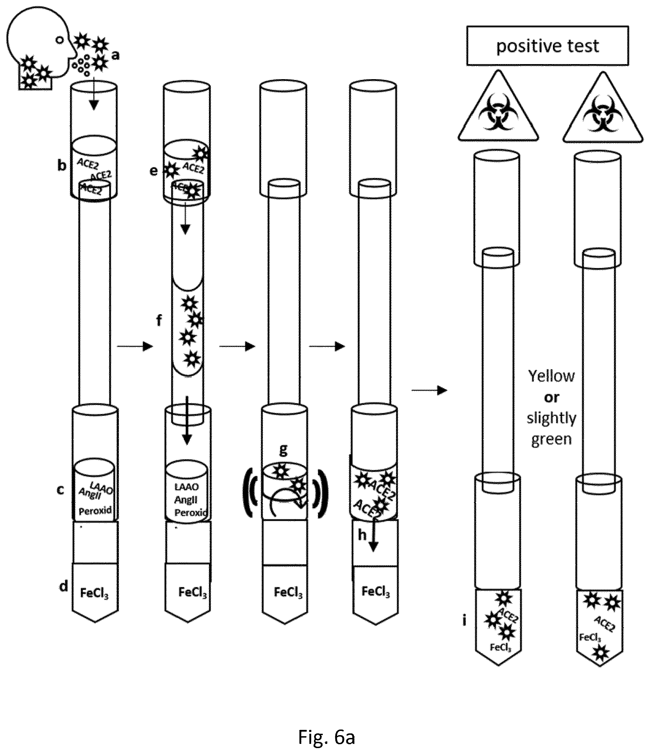

**Fig. 6a****:** Liquid assay without lateral flow technique using the phenylpyruvate - FeCl₃ strategy. A SARS-CoV-2 positive test result is shown. (a) An infected sample is applied into the application chamber. (b) The application chamber is locked until the sample is applied. The application chamber contains the ACE2 solution without the substrate (Ang II) (c) Reaction chamber. The application chamber is unlocked to enable the mixture of sample and reaction-mix in the reaction chamber. The reaction chamber contains all other remaining assay compounds except the FeCl₃ solution. The reaction chamber, containing initially only the pure reaction-mix, is also locked to prevent leakage. (d) Detection chamber. The detection chamber contains the FeCl₃ solution. (e) The test sample (from the patient) containing the virus is mixed with the ACE2 solution and incubated for about 5 min to enable the binding process of the virus to its receptor (ACE2). (f) The application chamber is unlocked to enable the transfer of the ACE2/virus complex into the reaction chamber. (g) Shaking of the device enables the mixture of test sample and reaction-mix. (h) The mixture is incubated for additional 5-10 minutes to enable the "theoretically possible enzyme-substrate reactions" (ACE2/Ang II and LAAO/Phe/Catalase). After incubation, the reaction chamber is unlocked to enable the transfer of the test sample into the detection chamber, containing the FeCl₃ solution. (i) Complete blockade of ACE2. Mixture of test sample, reaction mix and FeCl3 solution. The FeCl₃ solution remains yellow, since the protease function of ACE2 is blocked, - no phenylpyruvate is formed, - indicating a SARS-CoV-2 positive sample. (j) Partially blockade of ACE2. Mixture of test sample, reaction mix and FeCl₃ solution. The FeCl₃ solution becomes slightly bright green, since the protease function of ACE2 is partially blocked, - less phenylpyruvate is formed, - also indicating a SARS-CoV-2 positive sample. The color development of the positive control is much more intensive green than the slightly bright green of solution containing a "partially blocked" ACE2.

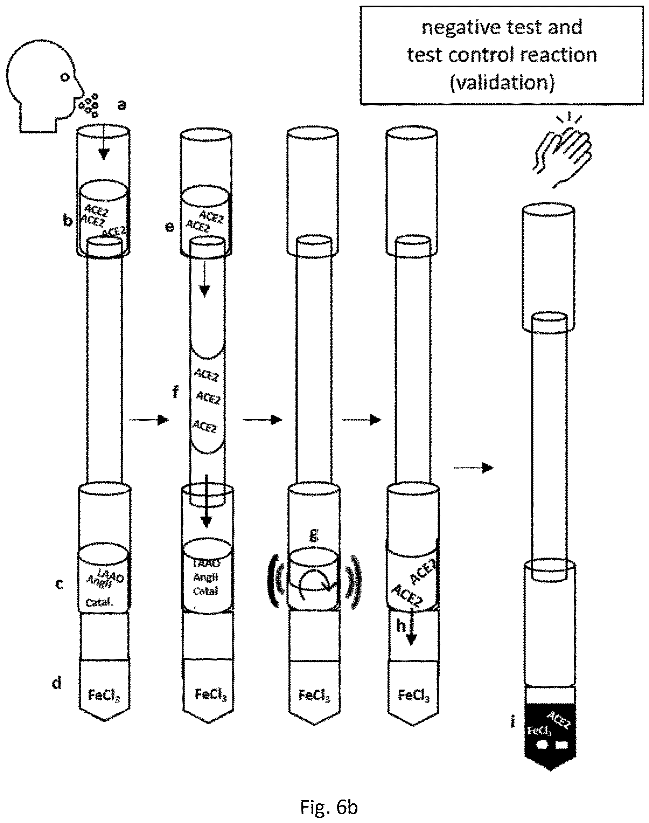

**Fig. 6b****:** Liquid without lateral flow technique using the phenylpyruvate - FeCl₃ strategy. A SARS-CoV-2 negative test result is shown (as well as the positive control). (a) A non-infected sample is applied into the application chamber. (b) The application chamber is locked until the sample is applied. The application chamber contains the ACE2 solution without the substrate (Ang II) (c) Reaction chamber. The application chamber is unlocked to enable the mixture of sample and reaction-mix in the reaction chamber. The reaction chamber contains all other remaining assay compounds except the FeCl₃ solution. The reaction chamber, containing initially only the pure reaction-mix, is also locked to prevent leakage. (d) Detection chamber. The detection chamber contains the FeCl3 solution. (e) The test sample (from a Corona negative patient) containing no virus is mixed with the ACE2 solution and incubated for about 5 min to enable the "binding process" of the virus to its receptor (ACE2) in case of a Corona positive sample. (f) The application chamber is unlocked to enable the transfer of the ACE2 solution into the reaction chamber. (g) Shaking of the device enables the mixture of test sample and reaction-mix. (h) The mixture is incubated for additional 5-10 minutes to enable the enzyme-substrate reactions" (ACE2/Ang II and LAAO/Phe/Catalase). After incubation, the reaction chamber is unlocked to enable the transfer of the test sample into the detection chamber, containing the FeCl₃ solution. (i) No blockade of ACE2. Mixture of test sample, reaction mix and FeCl₃ solution. The FeCl₃ solution becomes dark brown/green colored, since the protease function of ACE2 is fully intact, - subsequently phenylpyruvate is formed, - indicating a SARS-CoV-2 negative sample. The principle of a negative test sample and the positive control reaction (validation that the test was executed in a correct manner) is the same (always a dark colored brown/green solution). In case of the positive control reaction, buffer or a 0.9% NaCl solution is used instead of the test sample from the patient (gargle solution).

### Preferred embodiments:

1. A method for detecting a receptor binding virus in a sample, wherein said receptor exhibits enzymatic properties, comprising the steps of:
   a) incubating said sample with said receptor to form a sample-receptor mixture;
   b) contacting the mixture of step a) with a substrate catalyzable by said receptor;
   c) determining if said substrate is converted by said receptor; and
   d) detecting a receptor binding virus in said sample if said substrate is converted by said receptor to a lower extent compared to a reference sample.
2. The method of embodiment 1, wherein said reference sample is a sample substantially free of said receptor binding virus.
3. The method of embodiment 1 or 2, wherein said receptor is selected from the group consisting of angiotensin-converting enzyme 2 (ACE2), dipeptidyl peptidase-4 (DPP4), aminopeptidase N (AP-N), complement receptor type 1 (CR1), carboxypeptidase D and insulin-degrading enzyme (IDE).
4. The method of any one of embodiments 1 to 3, wherein said receptor binding virus is selected from the group consisting of a severe acute respiratory syndrome (SARS)-related coronavirus, Middle East respiratory syndrome (MERS)-related coronavirus, human coronavirus 229 E, transmissible gastroenteritis coronavirus, porcine epidemic diarrhea virus, human gammaherpesvirus 4, avian hepatitis B virus and human alphaherpesvirus 3 (HHV-3).
5. The method of any one of embodiments 1 to 4, wherein said receptor is ACE2 and said virus is a severe acute respiratory syndrome (SARS)-related coronavirus.
6. The method of embodiment 5, wherein said coronavirus is SARS-CoV-2.
7. The method of any one of embodiments 1 to 6, wherein the conversion of said substrate is determined colorimetrically, chromatographically, or fluorometrically.
8. The method of any one of embodiments 1 to 7, wherein said substrate is converted to at least one first product, wherein said at least one first product is detected by converting said at least one first product to at least one second product by a second enzyme, wherein said at least one second product is detected colorimetrically, chromatographically, or fluorometrically.
9. The method of embodiment 7 or 8, wherein the conversion of said substrate or said at least one second product is determined colorimetrically by the colour change of a redox agent or a pH indicator.
10. The method of any one of embodiments 1 to 9, wherein said substrate is a peptide comprising or consisting of the amino acid sequence (X₁)ₘDRVYIHP(X₂)ₙ (SEQ ID No. 1), wherein X₁ is an N-terminal tag, preferably selected from the group consisting of a cholesteryl compound, an aromatic compound, a heterocyclic compound and a heteroaromatic compound, and wherein X₂ is selected from the group consisting of phenylalanine (F), a fluorescent label and a chromophoric label, preferably para-nitroaniline, and wherein m is 0 or 1 and n is 0 or 1.
11. The method of embodiment 10, wherein said substrate is a peptide comprising or consisting of amino acid sequence DRVYIHPF (SEQ ID No. 2).
12. The method of any one of embodiments 8 to 11, wherein said at least one first product comprises phenylalanine.
13. The method of any one of embodiments 8 to 12, wherein said second enzyme is a phenylalanine processing enzyme (PPE), preferably selected from the group consisting of L-amino acid deaminase (LAAD), L-amino acid oxidase (LAAO), phenylalanine ammonia lyase (PAL) and L-amino acid dehydrogenase.
14. The method of any one of embodiments 8 to 13, wherein said second product comprises ammonia and/or phenylpyruvate and/or hydrogen peroxide.
15. The method of embodiment 14, wherein said ammonia is detected by the colour change of a pH indicator, wherein said pH indicator is preferably a triarylmethane dye, preferably selected from the group consisting of bromothymol blue and bromocresol purple.
16. The method of embodiment 14, wherein said phenylpyruvate is detected by the colour change of a redox agent, wherein said redox agent is preferably FeCl₃.
17. The method of embodiment 14, wherein said hydrogen peroxide is detected by the colour change of a compound which is oxidized by hydrogen peroxide in the presence of a peroxidase, wherein said compound is preferably 3,3',5,5'-tetramethylbenzidine.
18. The method of any one of embodiments 1 to 17, wherein said method is performed in liquid phase and/or on a solid phase.
19. The method of any one of embodiments 8 to 18, wherein said first product and said at least one second product is formed in liquid phase, and wherein said at least one second product is detected using a solid support comprising a redox agent and optionally a trap agent, wherein said solid support is preferably a filter strip.
20. The method of embodiments 19, wherein said redox agent is FeCl₃ and said trap agent is a quaternary ammonium compound.
21. A kit for detecting a receptor binding virus in a sample comprising:
   a) a receptor binding said virus, wherein said receptor exhibits enzymatic properties;
   b) a substrate catalyzable by said receptor;
   c) optionally a second enzyme converting the product of the conversion of said substrate by said receptor;
   d) at least one compound undergoing a colour change if said substrate is converted by said receptor.
22. The kit of embodiment 21, wherein said receptor binding virus is SARS-CoV-2, and said receptor is ACE2.
23. The kit of embodiment 21 or 22, wherein said compound undergoing a colour change is a redox agent positioned on a solid support, wherein said redox agent is preferably FeCl₃.
24. The kit of embodiment 23, wherein said solid support further comprises a trap agent, wherein said trap agent is preferably a quaternary ammonium compound.
25. A test strip for detecting a receptor binding virus in a sample, comprising a solid support comprising said receptor, a substrate catalyzable by said receptor, a compound undergoing a colour change if said substrate is converted by said receptor, and optionally a second enzyme converting the product of the conversion of said substrate by said receptor.
26. The test strip of embodiment 25, wherein said receptor binding virus is SARS-CoV-2, and said receptor is ACE2.
27. The test strip of embodiment 25 or 26, wherein said compound undergoing a colour change is a redox agent, preferably FeCl₃.
28. The test strip of any one of embodiments 25 to 27, wherein said test strip is a lateral flow test strip.

### EXAMPLES

### Example 1: Detection of SARS-CoV-2 based on pH value strategy

ACE2 converts Ang II, an octapeptide with C-terminal Phe, to the heptapeptide Ang (1-7) and the free amino acid residue phenylalanine (Phe). Subsequently, the released Phe can be used as a substrate from varying enzymes as described herein, that all form different α-keto acids like phenylpyruvate (phenylpyruvic acid) and ammonia/ammonium, H₂O₂ or NADH. The first option is to use a NH₃ producing enzyme (e.g. LAAD, LAAO or PAL). The addition of a suitable pH indicator (e.g. bromocresol red or bromothymol blue) to the reaction-mix makes the progress of the substrate (Phe) conversion visible. The NH₃ released during the enzyme reaction shifts the pH value into the alkaline range, which means that the added pH indicator changes its colour.

In the first step of the assay, the synthetic octapeptide (Ang II) was converted by ACE2, whereby the products Ang (1-7) and the free amino acid phenylalanine were formed. The free Phe was used as a substrate by phenylalanine converting enzymes (see Table 1) and converted to NH₃ and the corresponding α-keto acid (phenylpyruvate or trans cinnamic acid).

**Table 1: Phenylalanine processing enzymes (PPEs) used for the 2^{nd} step of the assay.**

| Enzyme | UniProt number | products | source |
|---|---|---|---|
| L-amino acid Deaminase (LAAD) | B4EZ74 | phenylpyruvate + NH₃ | expressed in E. coli |
| L-amino acid Oxidase (LAAO) | P56742 | phenylpyruvate + H₂O₂+ NH₃ | Merck |
| Phenylalanine ammonia lyase (PAL) | V5TFQ0 | trans-cinnamic acid + NH₃ | Merck |
| L-amino acid Dehydrogenase (LAADH) | P97014 | phenylpyruvate + NADH + NH₄⁺ | Merck |

The release of NH₃ caused a pH shift into the alkaline range, whereby the pH indicator added at the beginning of the enzyme-substrate reaction changed the colour of the reaction solution. This indicates that the tested sample does not contain any coronavirus, since the 1^{st} enzyme reaction (ACE2) can take place and thus provides the substrate (Phe) for the 2^{nd} enzyme reaction. If the sample is positive, the ACE2 activity is blocked or strong decelerated through binding of the virus to ACE2. This results in blocked or severely limited conversion of the physiological substrate (the synthetic octapeptide Ang II) and thus, the release of phenylalanine. This means that there is no substrate (Phe) for the second enzyme reaction (phenylalanine deaminase) available, - the 2^{nd} reaction cannot take place and thus no NH₃ is formed. The pH indicator does not change its colour, the solution retains its original colour, indicating a positive test result. The principle of this assay is shown schematically in Fig. 1.

### Optimization of 2^{nd} enzyme reaction:

For the 2^{nd} enzyme reaction different pH-indicators (Table 2) and buffers with various pH-values and salt concentrations (Table 3) were investigated. All buffers were prepared in the range from 1 mM to 10 mM.

**Table 2: Used pH-indicators**

| | **pH-indicator** |
|---|---|
| A | Phenolphtalein |
| B | Bromothymol Blue |
| C | Bromophenolred |
| D | Phenolred |
| E | Bromocresol purple |

**Table 3: Used buffers**

| **No.** | **pH value** | **buffer** |
|---|---|---|
| 1 | 5 | Citrate - Phosphate |
| 2 | 5.5 | Citrate - Phosphate |
| 3 | 6 | Citrate - Phosphate |
| 4 | 6.2 | Sodium - Phosphate |
| 5 | 6.5 | Sodium - Phosphate |
| 6 | 7.0 | Sodium - Phosphate |
| 7 | 7.3 | Tris |
| 8 | 7.5 | Tris |
| 9 | 7.7 | Tris |
| 10 | 8.0 | Tris |
| 11 | 8.5 | Tris |
| 12 | 9.0 | Tris |

The formation of the phenylalanine (substrate) that takes place within the 1^{st} enzyme reaction (ACE2) was simulated by the direct addition of Phe (different concentrations). Beyond that, various PPEs (Table 1) with different enzyme concentrations (U/ml) were chosen to investigate the formation of NH₃ and the resulting pH-shift.

Indicator B (Bromothymol blue) was selected for further applications since there are three colour-shifts between pH 5.5 and pH 7.7 from yellow to green to turquoise to blue. For further applications, 1mM to 5 mM buffers were selected since the colour-shift is achieved easier using a buffer with lower buffer-capacity and no loss of colour intensity was observed when compared to 10 mM buffers.

For the conversion of phenylalanine to the corresponding α-keto acids and NH₃ following PPEs were used:
- Phenylalanine ammonia lyase (PAL); stock solution: 0.5 U/ml
- L-Amino Acid Oxidase (LAAO); stock solution: 2 U/ml
- Catalase, stock solution 5000 U/ml; If LAAO was used as enzyme, also Catalase was added to the reaction, since H₂O₂ is generated as by-product through this enzyme (see: Table 1). The addition of Catalase prevents the loss of LAAO activity due to the formation of H₂O₂.

The concentration of Phe (substrate) was analysed in the range of 1 mM - 20 mM. For the enzyme assays a final concentration of 5 mM or 10 mM Phe was used.

For the establishment of the enzyme assay usually buffers with pH=6, pH=6.5 and pH=7.0 were used. Bromothymol Blue was dissolved in buffer with a final concentration of 1 mg/ml.

All assays were carried out as microtiter plate assays (MTP assays) with a final volume of 100 µl. Negative controls were performed using the same volume of buffer or water like in the enzyme-substrate reaction, instead of the corresponding enzyme or substrate, respectively.

Best results were obtained using the L-amino acid Oxidase (LAAO). However, the colour change compared to the negative control was of rather low intensity. The main reason for this is likely that the enzymes itself are lyophilized together with their buffer salts (e.g. acetate-buffer).

### Example 2: Detection of SARS-CoV-2 based on redox agent strategy

Applying this strategy, the addition of a pH indicator is not necessary. In the first step of the assay (using the protease function of ACE2 which is identical for both strategies), the synthetic octapeptide (Ang II) is converted by ACE2, whereby the heptapeptide Ang (1-7) and the free amino acid phenylalanine are formed, which is used as substrate by the PPE and converted to phenylpyruvate and the corresponding by-products which are shown in Table 1. In the next step, the "Fölling Probe" is used for the detection of phenylpyruvate. A FeCl₃ solution (10%) is added to the phenylpyruvate (colourless) formed during the phenylalanine deaminase reaction, which reduces the Fe^{III+} + to Fe^{II+}. The reduction of Fe^{III+} causes the colouring of the solution from colourless to green. This indicates that the tested sample does not contain any coronavirus, as the 1^{st} enzyme reaction (ACE2) takes place and thus provides the substrate (Phe) for the 2^{nd} enzyme reaction.

As already described in Example 1 (pH value), if the sample is positive, ACE2 is blocked or its protease activity is strongly decelerated caused by steric hinderance through binding of the virus. The physiological substrate (the octapeptide; Ang II) binds to a very low extent or not at all to the active site for the protease activity. As a result, the substrate (Phe) for the 2^{nd} enzyme reaction (PPE) is not formed or only formed to a minor extent. Hence, ideally the PPE-reaction cannot take place or the formation of phenylpyruvate is massively reduced and temporally strongly delayed to a time-point outside the "detection window". If no phenylpyruvate is formed, the FeCl₃ cannot react (no reduction) and the iron remains trivalent (Fe^{III+}) . Since no Fe^{II+} - phenylpyruvate complex is formed or the formation takes a long period of time (outside the detection window), the reaction solution remains colourless, - indicating a positive test result. The assay is shown schematically in Fig. 2.

### Optimization of 2^{nd} enzyme reaction:

For the 2^{nd} enzyme reaction two different enzymes were chosen to produce phenylpyruvate. These are the L-Amino Acid Oxidase (LAAO) and the L-Amino Acid Deaminase (LAAD). The latter enzyme is not commercially available; thus, the corresponding gene termed aad from Proteus mirabilis (GeneBank U35383.1) was heterologous expressed in E. coli BL21 (DE3) using a pET32b+ expression vector. Additionally, a N- or C-terminal His-tag is attached to the gene. The aad gene was fused to a maltose-binding protein (MBP) to improve the solubility of the Aad (LAAD) protein which is known as slightly soluble membrane protein. Primarily, the best assay conditions for the FeCl₃ based strategy were figured out by using directly different concentrations of the phenylpyruvate, the product of the LAAO deamination reaction of Phe. Subsequently, the optimal enzyme- (LAAO) and substrate (Phe)-concentration as well as buffers (50 mM and 100 mM) with pH values ranging from pH 5.5 to pH 9.0, were investigated. At least the optimal Catalase concentration was determined since the LAAO forms H₂O₂ as by-product to phenylpyruvate and NH₃. The Catalase is used to remove the H₂O₂ which accumulates during the reaction in the same extent as the Phe is converted to phenylpyruvate and NH₃. An increasing H₂O₂ concentration disturbs the LAAO reaction drastically, finally leading to failed detection of phenylpyruvate by FeCl₃. The appropriate FeCl₃ concentration was fixed with 10%.

To check the influence of the pH value on the colour development through the addition of 10% FeCl₃ - phenylpyruvate (final concentration 5mM) - the product of the 2^{nd} reaction was directly added to the assay. For further experiments, a working range of pH 6.5 to 7.5 was selected since both enzyme reactions (ACE2 and PPE) as well as the colouring reaction worked well within this pH area.

To determine the optimal enzyme concentration using LAAO, enzyme concentrations in the range from 0.8 to 0.01 U/ml were selected. The deamination reactions of phenylalanine using the LAAO were carried out with 100 mM sodium phosphate buffer pH 7 and 5 mM Phe (final concentration). Additionally, Catalase was added to the assays to prevent the formation of H₂O₂ which is a by-product of the phenylalanine-LAAO-reaction and disturbs the enzyme-substrate reaction(final catalase concentration: 500 U/ml (0.1 mg/mL)). Negative controls were performed with the same volume of pure H₂O instead of LAAO. Enzyme substrate reactions were incubated in the range of 2 to 10 minutes. Subsequently, 10 µl of FeCl₃ (10%) were added to each well to start the colour detection. The final assay volume was 100 µl. The production of phenylpyruvate through deamination of Phe by the LAAO was shown as green coloured wells. Negative controls that contain no LAAO enzyme remained colourless since no phenylpyruvate was formed. The best result (clearest colour development) was obtained for a LAAO concentration of 0.4 U/ml.

For further applications, the LAAO concentration of 0.4 U/ml (final assay concentration) was chosen.

### Addition of Catalase to improve the colour development:

The addition of Catalase prevents the accumulation of H₂O₂ that disturbs the enzyme reaction and improves the development of the green colour. Without Catalase a bleaching effect of the green colour appears after addition of FeCl₃. The optimal amount of Catalase (U/ml) was evaluated which should be added to the 2^{nd} enzyme reaction (PPE): This assay was carried out with 100 mM sodium phosphate buffer pH 6.5 and 7.0. The final substrate concentrations (Phe) were 30, 10 and 5 mM. The final LAAO concertation was 0.4 U/ml. Without the addition of catalase, bleaching of the green colour was observed after 1-5 min. In the presence of catalase, the green colour remained stable (independent of the substrate concentration). For negative controls (containing no LAAO), no colour development was observed. FeCl₃ (10%) was added to each well after 5 min incubation time to start the colour detection. The final assay volume was 100 µl.

The final Catalase concentration for the enzyme assay was evaluated in the range of 500 - 0.1 U/ml. The optimal Catalase concentration was found to be 50-150 U/ml, preferably 100 U/mL. Increase of the Catalase concentration accelerates the development of the green colour.

### Determination of the optimal substrate (Phe) concentration:

The minimal detectable substrate concentration (0.25 mM - 5 mM) that is required for the detection with FeCl₃ was figured out using phenylalanine as substrate for the LAAO. Since the 1^{st} enzyme reaction (ACE2) produces the substrate (Phe) for the 2^{nd} (PPE) enzyme reaction it is necessary to evaluate the minimal Phe-concentration that is required to be converted into phenylpyruvate which is detected by the final addition of FeCl₃.

This assay was carried out with 100 mM sodium phosphate buffer pH 6.5 The final substrate concentrations (Phe) ranged from 5 mM to 0.25 mM. The final LAAO concertation was 0.4 U/ml. Negative controls contained no Phe. Catalase was added at a concentration of 50 U/ml (final assay concentrations). FeCl₃ (10%) was added to each well after 5 min incubation time to start the colour detection. The final assay volume was 100 µl.

Best substrate concentrations range were found to be between 1 mM to 5 mM.

### Example 3: Testing strategy based on the determination of H₂O₂ formation

Beside the detection of phenylpyruvate through FeCl₃, a second method was established by using the formed H₂O₂ from the LAAO reaction (see Table 1) for the oxidation of chromogenic compounds. This oxidation process leads to a colour change of the reaction solution from colourless to coloured. In comparison to the FeCl₃ strategy, no Catalase is added to the enzyme assay to destroy the generated H₂O₂. In contrast, the opposite effect is utilized for the visualization of the LAAO reaction. A Peroxidase is added to the reaction solution that utilizes the formed H₂O₂ to oxidize chemical substances like Guaiacol, 3,4-diaminobenzoic acid, tetramethylbenzidine (TMB) and derivates thereof.

First, the LAAO reaction (PPE) was observed by adding Peroxidase and Guaiacol. The assay was carried out with 50 mM sodium phosphate buffer pH 6.5. The final substrate concentration (Phe) was 5mM. The final LAAO concertation was 0.8 U/ml, the Peroxidase concentration 16 U/ml. The Guaiacol concentration was 80 mM. The reaction (LAAO/Phe - Peroxidase/H₂O₂/Guaiacol) showed the development of red colour. Negative controls contained no LAAO, or no Peroxidase or no Guaiacol and displayed no colour development. The colour development took place after 1-5 min incubation time. The final assay volume was 100 µl.

Beside the detection method through the oxidation of Guaiacol, a second method using 3,4-diaminobenzoic acid (DABA) as substrate was established. The principle of this detection method is identical to the Peroxidase/H₂O₂/Guaiacol system. The H₂O₂ generated by the LAAO reaction is subsequently used by the Peroxidase for the oxidation of 3,4-diaminobenzoic acid instead of Guaiacol. The oxidation process also leads to a colour change of the reaction solution, particularly from colourless to brown.

In addition, a third method using Peroxidase and tetramethylbenzidine (TMB) for detection was established. The principle of this detection method is identical to the Peroxidase/H₂O₂/Guaiacol and Peroxidase/H₂O₂/DABA systems.

### Example 4: Influence of physiological factors (salvia) on the result of the PPE-reaction

To ensure that compounds or metabolites contained in the human saliva do not lead to an unwanted colour change and thus causing a false negative result, a saliva sample-mix from healthy volunteers was used as test sample. In comparison to the PPE-reaction (FeCl₃- and Peroxidase/H₂O₂-strategy), no colour change occurs, since no LAAO is available for the deamination of Phe to phenylpyruvate and H₂O₂ which were responsible for the colour development. The 1^{st} positive control was performed by adding LAAO, - the 2^{nd} one by adding DABA and Peroxidase, to the assay reaction. As expected, the addition of salvia led to no colour change - in contrast, the positive controls showed the expected colour change from colourless to green or brown, respectively.

### Example 5: "Lateral flow test strip" (solid phase assay) for pH value strategy

Fig. 3a and 3b show a scheme of a lateral flow test strip based on the pH value strategy (see Example 1). For the sample application, the samples (smear) which were previously obtained by swabbing, were resuspended in the smallest possible volume (buffer or 0.9% NaCl). A few microliters (e.g.100 µl) of the samples were transferred to the application area, which starts the test-reaction. The two detection tracks must be designed in such a way that no liquid transfer or exchange between the two tracks becomes possible. The left detection track is used for sample analysis (test track, the right lane is the positive control. This means that while a defined sample volume is applied to the left detection path, the same volume of pure buffer or 0.9% NaCl should be applied to the right track.

The pH indicator area on the right detection lane (Fig. 3a) should always turn blue. If not, the entire test is invalid. In contrast, there are two possibilities for the left lane. Either the pH indicator area also turns blue or it remains yellow or yellow/green (= pH value at approx. ∼ 6.0 - 6.5). If the left pH indicator area turns blue, the corresponding sample that was applied onto the device is negative, since the entire enzyme cascade takes place and finally NH₃ gets released. If the pH indicator area remains yellow, which is also the originally colour of the detection area, the applied sample is positive, since the enzyme cascade is interrupted. ACE2 is bound by virus particles and cannot produce substrate (phenylalanine) for the 2^{nd} enzyme reaction (PPE). The second enzyme reaction does not take place - no NH₃ is formed-, finally the pH indicator area remains yellow.

For the evaluation of the test it is necessary to compare the "sample lane" (left side) with the "positive control lane" (right side). If the same colour appears in both detection windows, the applied sample is "NEGATIVE". If different colours appear in both detection windows (left: YELLOW or YELLOW/GREEN / right: BLUE), the applied sample is "POSITIVE". Fig. 3b shows the plot of a positive and negative test. The variant A (left) displays the in-vitro diagnostic (IVD)-device in case of a SARS-CoV-2-positive, the variant B (right) in case of a SARS-CoV-2-negative test result (SARS-CoV-2 sample analysis).

### Example 6: Combination of solid phase (lateral flow) and liquid phase enzyme-assay for pH value strategy

The combination of a liquid assay and a solid phase detection mode (lateral flow) is structured as follows. Both enzyme reactions, the ACE2 and the PPE reaction are performed simultaneously in the same reaction tube. In the first step of the assay, the synthetic octapeptide (Ang II) is converted by ACE2, whereby the products Ang (1-7) and the free aa phenylalanine are formed. The free Phe is used as a substrate by PPEs (phenylalanine converting enzymes; see: Table 1) and converted to NH₃ and the corresponding α-keto acid (phenylpyruvate or trans cinnamic acid) and +/- H₂O₂ or NADH (depending on the used type of PPE). The release of NH₃ causes a pH shift into the alkaline range, whereby the pH indicator changes its colour. After a few minutes' reaction time (5-20 min), the solid phase (lateral flow device) and the liquid phase (ACE2 and PPE reaction) are brought into physical contact to each other to start the lateral flow of the reaction directed to the pH-responsive area. The pH indicator is fixed onto the filter paper or the filter paper itself contains a pH-sensitive zone at the upper end of the filter strip (lateral flow device). A colour change within the pH-responsive area/zone indicates that the tested sample does not contain any coronavirus, since the 1^{st} enzyme reaction (ACE2) can take place and thus provides the substrate (Phe) for the 2nd enzyme reaction. If the sample is positive, the ACE2 activity is blocked or strong decelerated through binding of the virus to ACE2. This results in blocked or severely limited conversion of the physiological substrate (the synthetic octapeptide Ang II) and thus, the release of phenylalanine. This means that there is no substrate (Phe) for the second enzyme reaction (a phenylalanine processing enzyme) available, - the 2^{nd} reaction cannot take place and thus no NH₃ is formed. The fixed pH indicator or the pH-sensitive area itself does not change its colour, the pH responsive paper retains its original colour, indicating a positive test result.

### Example 7: "Lateral flow test strip" (solid phase assay) for redox agent strategy (FeCl₃ or phenolic compound)

4a shows a scheme of a lateral flow test strip based on the FeCl₃ strategy (see Example 2, detection of phenylpyruvate by FeCl₃). Samples were applied as described in Example 5.

For the evaluation of the test, it is necessary to compare the "sample lane" (left side) with the "positive control lane" (right side). If the brown/green band appears in both detection windows, the applied sample is "NEGATIVE". If the band only appears in the detection window of the right lane (positive control), but not in the detection window of the left lane, (left: white area - no band / right: brown/green band), the applied sample is "POSITIVE". Fig. 4b shows the plot of the prototype. The variant A (left) displays the IVD-device in case of a SARS-CoV-2-positive, the variant B (right) in case of a SARS-CoV-2-negative test result.

A further lateral flow test strip was designed based on the detection of H₂O₂ with a chromogenic compound (see Example 3). In this case, LAAD was used as an enzyme, which produces H₂O₂ in case the substrate is converted by LAAD. If H₂O₂ is formed (negative sample), it thereafter oxidizes a phenolic compound to give a coloured band on the test strip. In case no H₂O₂ is formed, the sample is positive.

### Example 8: Combination of solid phase (lateral flow) and liquid phase enzyme-assay for redox agent strategy (FeCl₃)

The combination of a liquid assay and a solid phase detection mode (lateral flow) is structured as follows. Both enzyme reactions, the ACE2 and the PPE reaction are performed simultaneously in the same reaction tube, carried out as liquid assay. In the first step of the assay, the synthetic octapeptide (Ang II) is converted by ACE2, whereby the products Ang (1-7) and the free aa phenylalanine are formed. The free Phe is subsequently used as a substrate by PPEs (phenylalanine converting enzymes; see: Table 1) and converted to phenylpyruvate and NH₃ +/- H₂O₂ or NADH (depending on the used type of PPE). Catalase is added to the assay to eliminate the formed H₂O₂ which disturbs the enzyme cascade. The formed phenylpyruvate can be detected by the addition of the yellow colored FeCl₃, that changes its color to green/brown. This visualization step is realized with a lateral flow device.

After a few minutes' reaction time (5-20 min), the solid phase (lateral flow device) and the liquid phase (ACE2 and PPE reaction) are brought into physical contact to each other. The lateral flow device consists of the detection area containing the FeCl₃ as well as the tetra-n-octylammonium bromide trap. The trap is fixed onto the filter paper as thin line above the applied FeCl₃. The development of a brown/green band within the trap-area/zone indicates that the tested sample does not contain any coronavirus, since the 1^{st} enzyme reaction (ACE2) can take place and thus provides the substrate (Phe) for the 2nd enzyme reaction. If the sample is positive, the ACE2 activity is blocked or strong decelerated through binding of the virus to ACE2. This results in blocked or severely limited conversion of the physiological substrate (the synthetic octapeptide Ang II) and thus, the release of phenylalanine. This means that there is no substrate (Phe) for the second enzyme reaction (a phenylalanine processing enzyme) available, - the 2^{nd} reaction cannot take place and thus no phenylpyruvate is formed. The trap-area (detection window) remains empty, - no brown/green band is visible, or the formation of the band is temporally outside of the detection window. Beyond that, the formed band can be much weaker than those of the positive control reaction. The schemes of the combined assays (liquid and solid) are shown in Figures 5a and 5b.

### Example 9: Combination of solid phase (lateral flow) and liquid phase enzyme-assay for redox agent strategy (peroxidase-oxidizable substrate)

This combination of a liquid assay and a solid phase detection mode (lateral flow) is structured as follows. Both enzyme reactions, the ACE2 and the PPE reaction are performed simultaneously in the same reaction tube, carried out as liquid assay. In the first step of the assay, the synthetic octapeptide (Ang II) is converted by ACE2, whereby the products Ang (1-7) and the free aa phenylalanine are formed. The free Phe is subsequently used as a substrate by PPEs (phenylalanine converting enzymes; see: Table 1) and converted to phenylpyruvate and NH₃ +/- H₂O₂ or NADH (depending on the used type of PPE).

In case of L-amino acid oxidase (LAAO) the formed H₂O₂ can be used for the oxidation of a chromogenic substrate by peroxidase (e.g. Guaiacol, 3,- 4,- diamino benzoic acid (DABA) or 3,3',5,5'-tetramethylbenzidine (TMB)).

The oxidation of Guaiacol or DABA generates a colored band within the trapping zone of the filter stripe (lateral flow device), consisting of tetra-n-octylammonium bromide, which should be applied onto the filter paper (solid phase). After a few minutes' reaction time (5-20 min), the solid phase (lateral flow device) and the liquid phase (ACE2 and PPE reaction) is brought into physical contact to each other. The lateral flow device consists of the detection area containing the applied tetra-n-octylammonium bromide trap and the peroxidase enzyme. The trap is fixed onto the filter paper as thin line above the applied peroxidase. As 2^{nd} variant, the peroxidase can be included into the liquid assay, whereas only the trap is applied onto the filter stripe. The development of a colored band within the trap-area/zone indicates that the tested sample does not contain any coronavirus, since the 1^{st} enzyme reaction (ACE2) can take place and thus provides the substrate (Phe) for the 2^{nd} enzyme reaction. One metabolite of the 2^{nd} enzyme reaction, the H₂O₂, can be used subsequently by the peroxidase to oxidize a chromogenic substrate which leads to the colored band within the trapping area. If the sample is positive, the ACE2 activity is blocked or strong decelerated through binding of the virus to ACE2. This results in blocked or severely limited conversion of the physiological substrate (the synthetic octapeptide Ang II) and thus, the release of phenylalanine. This means that there is no substrate (Phe) for the second enzyme reaction (a phenylalanine processing enzyme) available, - the 2^{nd} reaction cannot take place and thus, subsequent no H₂O₂ is formed for the final peroxidase reaction. The trap-area (detection window) remains empty, - no chromogenic substrate is visualized through oxidation, or the formation of the colored band is temporally outside of the detection window. Beyond that, the formed band can be much weaker than those of the positive control reaction. The principle of the combined assays (liquid and solid) is the same as shown in Figures 5a and 5b. The used metabolites, H₂O₂ instead of phenylpyruvate and an additional enzyme (peroxidase) as well as an appropriate oxidizable chromogenic substrate, are integrated in the detection system.

In case of using the peroxidase/TMB detection system, peroxidase and TMB are provided on the filter strip and the liquid phase containing the reaction products is dropped onto the filter strip. In this case, no trap agent is needed.

### Example 10: Liquid phase enzyme-assay for redox agent strategy (FeCl₃)

This detection mode based on the liquid assay without lateral flow technique is structured as follows. Both enzyme reactions, the ACE2 and the PPE reaction are performed simultaneously in the same reaction tube, carried out as liquid assay. In the first step of the assay, the synthetic octapeptide (Ang II) is converted by ACE2, whereby the products Ang (1-7) and the free aa phenylalanine are formed. The free Phe is subsequently used as a substrate by PPEs (phenylalanine converting enzymes; see: Table 1) and converted to phenylpyruvate and NH₃ +/- H₂O₂ or NADH (depending on the used type of PPE). Catalase is added to the assay to eliminate the formed H₂O₂ which disturbs the enzyme cascade. The formed phenylpyruvate can be detected by the addition of the yellow colored FeCl₃, that changes its color to dark green/brown. This evaluation step is realized by comparison of the tube containing the positive control (always dark green/brown colored) and the test sample (yellow or bright green for positive sample). After a few minutes' reaction time (5 min) of ACE2 with the test sample (without the Ang II substrate), to ensure the blocking/partially blocking of the protease activity of ACE2 by the virus in case of a Corona-infection, this mixture (ACE2 or ACE2/virus) is brought into physical contact with the remaining assay compounds (e.g. Ang II, LAAO, Catalase). After an additional incubation time of about 5 minutes, in a final step the FeCl₃ solution is mixed with the test solution as well as the positive control reaction. The development of a dark brown/green color of the test-sample indicates that the tested sample does not contain any Coronavirus, since the 1^{st} enzyme reaction (ACE2) can take place and thus provides the substrate (Phe) for the 2nd enzyme reaction. If the sample is positive, the ACE2 protease activity is completely or partially blocked through binding of the virus to ACE2. This results in blocked or severely limited conversion of the physiological substrate (the synthetic octapeptide Ang II) and thus, the release of phenylalanine. This means that there is no or very less substrate (Phe) for the second enzyme reaction (a phenylalanine processing enzyme) available, - the 2^{nd} reaction cannot take place (or in a highly limited manner) and thus, no or very less phenylpyruvate is formed. The tube of the test sample remains yellow-, indicating a nearly complete inhibition of the ACE2 enzyme, or the color of the test sample becomes slightly bright green which suggests that the virus blocks the ACE2 enzyme partially in a lower extend. For this reason, the development of a slightly bright green colored solution should be prevented by shifting the formation of the color development outside of the detection window. If not possible, the comparison of the positive control reaction (which works in the same manner as a negative test sample) with corresponding test sample (from the patient) should lead to a clear distinguishable result like dark green/brown in contrast to slightly bright green. The working principle of a negative test sample and the positive control reaction (validation that the test was executed in a correct manner) is the same (always a dark colored brown/green solution). In case of the positive control reaction, buffer or a 0.9% NaCl solution is used instead of the test sample from the patient (gargle solution). The scheme for the liquid assays is shown in Figure 6a and 6b.

### Example 11: Liquid phase enzyme-assay for redox agent strategy (peroxidase-oxidizable substrate)

This detection mode based on the liquid assay without lateral flow technique is structured as follows. Both enzyme reactions, the ACE2 and the PPE reaction are performed simultaneously in the same reaction tube, carried out as liquid assay. In the first step of the assay, the synthetic octapeptide (Ang II) is converted by ACE2, whereby the products Ang (1-7) and the free aa phenylalanine are formed. The free Phe is subsequently used as a substrate by PPEs (phenylalanine converting enzymes; see: Table 2) and converted to phenylpyruvate and NH₃ +/- H₂O₂ or NADH (depending on the used type of PPE) .

In case of L-amino acid oxidase (LAAO) the formed H₂O₂ can be used for the oxidation of a chromogenic substrate by peroxidase (e.g. Guaiacol; DABA or TMB). The oxidation of a chromogenic substrate generates a colored solution. The development of a colored solution indicates that the tested sample does not contain any Coronavirus, since the 1^{st} enzyme reaction (ACE2) can take place and thus provides the substrate (Phe) for the 2nd enzyme reaction. One metabolite of the 2^{nd} enzyme reaction, the H₂O₂, can be used subsequently by the peroxidase to oxidize a chromogenic substrate which leads to the colored solution. If the sample is positive, the ACE2 activity is blocked or strong decelerated through binding of the virus to ACE2. This results in blocked or severely limited conversion of the physiological substrate (the synthetic octapeptide Ang II) and thus, the release of phenylalanine. This means that there is no or very less substrate (Phe) for the second enzyme reaction (a phenylalanine processing enzyme) available, - the 2^{nd} reaction cannot take place (or in a highly limited manner) and thus, subsequent no or very less H₂O₂ is formed for the final peroxidase detection reaction. The reaction mix does not change its color since no chromogenic substrate is visualized through oxidation. The development of a slightly colored solution, since the ACE2 enzyme is just partially blocked by the viral S1 spike protein, should be prevented by shifting the formation of the color development outside of the detection window. If not possible, the comparison of the positive control reaction (which works in the same manner as a negative test sample) with corresponding test sample (from the patient) should lead to a clear distinguishable result like an intensive colored solution in contrast to a weak colored solution. The working principle of a negative test sample and the positive control reaction (validation that the test was executed in a correct manner) is the same (always an intensive colored solution). The principle of the peroxidase based liquid assays is very similar to those shown in Figures 19a and 19b. In contrast, the used metabolites, H₂O₂ instead of phenylpyruvate and an additional enzyme (peroxidase) as well as an appropriate oxidizable chromogenic substrate, is integrated in the detection system. The test sample (from the patient) is mixed with the ACE2 enzyme in the application chamber and incubated for 5 minutes to enable the viral S1 spike protein, if available, the docking process to its receptor ACE2. In contrast to the FeCl₃ - phenylpyruvate based liquid assay, the reaction chamber of this assay variant contains all assay compounds (including the chromogenic substrate) and serves simultaneously as detection chamber. Since the transfer of the ACE2 or ACE2/virus-complex solution to the reaction chamber has occurred, the enzyme cascades starts immediately and H₂O₂ is generated. If the formed H₂O₂ is not consumed directly by the peroxidase to oxidize a chromogenic substrate it could be harmful for all participating enzymes used for the detection reaction. In summary it can be mentioned that the reaction chamber serves also as detection chamber as well as a second incubation step is obsolete, since the key-player of the enzyme cascade, the H₂O₂ must be converted immediately otherwise this circumstance could lead to a "false positive" test result.

## Claims

1. A method for detecting a receptor binding virus in a sample, wherein said receptor exhibits enzymatic properties, comprising the steps of:
a) incubating said sample with said receptor to form a sample-receptor mixture;
b) contacting the mixture of step a) with a substrate catalyzable by said receptor;
c) determining if said substrate is converted by said receptor; and
d) detecting a receptor binding virus in said sample if said substrate is converted by said receptor to a lower extent compared to a reference sample.

2. The method of claim 1, wherein said reference sample is a sample substantially free of said receptor binding virus.

3. The method of claim 1 or 2, wherein said receptor binding virus is selected from the group consisting of a severe acute respiratory syndrome (SARS)-related coronavirus, Middle East respiratory syndrome (MERS)-related coronavirus, human coronavirus 229 E, transmissible gastroenteritis coronavirus, porcine epidemic diarrhea virus, human gammaherpesvirus 4, avian hepatitis B virus and human alphaherpesvirus 3 (HHV-3).

4. The method of any one of claims 1 to 3, wherein said receptor is ACE2 and said virus is a severe acute respiratory syndrome (SARS)-related coronavirus, preferably SARS-CoV-2.

5. The method of any one of claims 1 to 4, wherein the conversion of said substrate is determined colorimetrically, chromatographically, or fluorometrically.

6. The method of any one of claims 1 to 5, wherein said substrate is converted to at least one first product, wherein said at least one first product is detected by converting said at least one first product to at least one second product by a second enzyme, wherein said at least one second product is detected colorimetrically, chromatographically, or fluorometrically.

7. The method of claim 5 or 6, wherein the conversion of said substrate or said at least one second product is determined colorimetrically by the colour change of a redox agent or a pH indicator.

8. The method of any one of claims 1 to 7, wherein said substrate is a peptide comprising or consisting of the amino acid sequence (X₁)ₘDRVYIHP(X₂)ₙ (SEQ ID No. 1), wherein X₁ is an N-terminal tag, preferably selected from the group consisting of a cholesteryl compound, an aromatic compound, a heterocyclic compound and a heteroaromatic compound, and wherein X₂ is selected from the group consisting of phenylalanine (F), a fluorescent label and a chromoph oric label, preferably para-nitroaniline, and wherein m is 0 or 1 and n is 0 or 1.

9. The method of any one of claims 6 to 8, wherein said at least one first product comprises phenylalanine.

10. The method of any one of claims 6 to 9, wherein said second enzyme is a phenylalanine processing enzyme (PPE), preferably selected from the group consisting of L-amino acid deaminase (LAAD), L-amino acid oxidase (LAAO), phenylalanine ammonia lyase (PAL) and L-amino acid dehydrogenase.

11. The method of any one of claims 6 to 10, wherein said second product comprises ammonia and/or phenylpyruvate and/or hydrogen peroxide.

12. The method of claim 11, wherein said phenylpyruvate is detected by the colour change of a redox agent, wherein said redox agent is preferably FeCl₃.

13. The method of claim 11, wherein said hydrogen peroxide is detected by the colour change of a compound which is oxidized by hydrogen peroxide in the presence of a peroxidase, wherein said compound is preferably 3,3',5,5'-tetramethylbenzidine.

14. The method of any one of claims 1 to 13, wherein said method is performed in liquid phase and/or on a solid phase.

15. A kit for detecting a receptor binding virus in a sample comprising:
a) a receptor binding said virus, wherein said receptor exhibits enzymatic properties;
b) a substrate catalyzable by said receptor;
c) optionally a second enzyme converting the product of the conversion of said substrate by said receptor;
d) at least one compound undergoing a colour change if said substrate is converted by said receptor.

16. The kit of claim 15, wherein said receptor binding virus is SARS-CoV-2, and said receptor is ACE2.

17. A test strip for detecting a receptor binding virus in a sample, comprising a solid support comprising said receptor, a substrate catalyzable by said receptor, a compound undergoing a colour change if said substrate is converted by said receptor, and optionally a second enzyme converting the product of the conversion of said substrate by said receptor.


SEQUENCE LISTING

<110>  Qualizyme Diagnostics GmbH & Co KG

<120>  Method for detecting a virus

<130>  27369-EP

<160>  2

<170>  PatentIn version 3.5

<210>  1
<211>  9
<212>  PRT
<213>  Artificial Sequence

<220>
<223>  Substrate catalyzable by ACE2



<220>
<221>  MISC_FEATURE
<222>  (1)..(1)
<223>  X is a or no N-terminal tag

<220>
<221>  MISC_FEATURE
<222>  (9)..(9)
<223>  X is a or no F, a or no fluorescent label or a or no chromophoric
       label

<400>  1

Xaa Asp Arg Val Tyr Ile His Pro Xaa
1               5



<210>  2
<211>  8
<212>  PRT
<213>  Angiotension 1-8 ("Ang II")

<400>  2

Asp Arg Val Tyr Ile His Pro Phe
1               5